# EUROPÄISCHE PATENTSCHRIFT

(11) **EP 1 904 404 B1**
(45) Veröffentlichungstag und Bekanntmachung des Hinweises auf die Patenterteilung: **25.02.2009**
(21) Anmeldenummer: 06764090.4
(22) Anmeldetag: 06.07.2006
(51) Int. Cl.: C01G 23/053, C01G 9/08, C01F 11/46, C09C 1/36, C09C 1/04, C09C 1/02, C09C 3/06, C09C 3/08, C09C 3/10, C09C 3/12, A61K 8/19, C08K 9/00

(54) **MARKIERTE ANORGANISCHE ADDITIVE**
MARKED INORGANIC ADDITIVES
ADDITIFS ANORGANIQUES MARQUES

(30) Priorität: 13.07.2005 DE 102005033208
(43) Veröffentlichungstag der Anmeldung: 02.04.2008
(73) Patentinhaber: Sachtleben Chemie GmbH, 47198 Duisburg (DE)
(72) Erfinder: EICKSCHEN, Ralf, 47445 Moers (DE); HOCKEN, Jörg, 40670 Meerbusch (DE); KASTNER, Jürgen, 44803 Bochum (DE)
(74) Vertreter: Uppena, Franz
(86) Internationale Anmeldenummer: PCT/EP2006/063942
(87) Internationale Veröffentlichungsnummer: WO 2007/006711

(56) Entgegenhaltungen:
- DE-A1- 10 200 802
- US-A- 5 286 468
- DATABASE BIOSIS [Online] BIOSCIENCES INFORMATION SERVICE, PHILADELPHIA, PA, US; 2004, ULBERTH FRANZ: "Analytical approaches for food authentication" XP002419952 Database accession no. PREV200500201614 & MITTEILUNGEN AUS LEBENSMITTELUNTERSUCHUNG UND HYGIENE, Bd. 95, Nr. 6, 2004, Seiten 561-572, ISSN: 1424-1307

## Beschreibung

Gegenstand der vorliegenden Erfindung sind markierte anorganische Additive, ein Verfahren zu deren Herstellung sowie deren Anwendung.

Insbesondere Gegenstand der vorliegenden Erfindung sind markierte anorganische Pigmente und Füllstoffe, ein Verfahren zu deren Herstellung sowie deren Anwendung.

Bei Lebensmitteln, Futtermitteln, Arzneimitteln aber auch bei vielen industriellen. Erzeugnissen besteht der Bedarf, einen eindeutigen Nachweis über die Herkunft zu haben. So ist es beispielsweise für Reklamationszwecke erforderlich nachzuweisen, von welchem Hersteller ein Rohstoff geliefert wurde. Umgekehrt wünschen sich die Lieferanten die Möglichkeit, sich durch eindeutigen Herkunftsnachweis vor Schadensersatzansprüchen zu schützen, die fälschlicherweise an sie gerichtet wurden. In der Medizin werden beispielsweise für das Einsatzgebiet Röntgenkontrastmittel spezielle synthetische Bariumsulfate eingesetzt. In Entwicklungsländern tauchen von Zeit zu Zeit Bariumsulfat-Produkte (Plagiate) für den genannten Einsatzzweck auf, die von minderer Qualität sind. Bei Verabreichung dieser Produkte kann eine Gesundheitsgefährdung der Patienten nicht ausgeschlossen werden. Es ist daher im Interesse der Hersteller hochqualitativer Produkte, die Verwendung der eigenen Produkte zweifelsfrei und in kleinsten Mengen nachweisen zu können, um sich notfalls vor unberechtigten Regressansprüchen schützen zu können.

Aus dem Stand der Technik ist es bekannt, die gelieferten Stoffe dadurch zu markieren, dass ihnen Stoffe mit einem erhöhten Anteil an schweren, stabilen Isotopen zugesetzt werden. In allen chemischen Verbindungen setzen sich die chemischen Elemente, aus denen die entsprechende Verbindung aufgebaut ist, aus verschiedenen stabilen Isotopen zusammen. Diese stabilen Isotope kommen in der Natur und damit auch in den zu markierenden Stoffen in natürlichen Verteilungsbreiten vor, so genannten "mittleren natürlichen Häufigkeiten" (Tabelle 1, Chiuz, 2005, 39, S. 93), so dass sehr hohe Konzentrationen der markierten Substanzen zugesetzt, insbesondere beigemischt werden müssen, um eine signifikante Abweichung zu bewirken.

**Tabelle 1: "mittlere natürliche Häufigkeiten" ausgewählter Isotope**

| **Element** | **Isotop** | | **Name** | **Standard** |
|---|---|---|---|---|
| | **Symbol** | **F[Atom-%]** | **(Abkürzung)** | **R** |
| Wasserstoff | ¹H | 99,9855 | Standard Mean | |
| | ²H | 0,0145 | Ocean Water | 0,00015576 |
| | | | (V-SMOW) | |
| Kohlenstoff | ¹²C | 98,892 | PEE Dee Belemnite | |
| | ¹³C | 1,1080 | (V-PDB) | 0,0011237 |
| Stickstoff | ¹⁴N | 99,6337 | Air Nitrogen | |
| | ¹⁵N | 0,3663 | (Air) | 0,0036765 |
| Sauerstoff | ¹⁶O | 99,7587 | Standard Mean | |
| | ¹⁷O | 0,0375 | Ocean Water | 0,0020052 |
| | ¹⁸O | 0,2039 | (V-SMOW) | |
| Schwefel | ³²S | 95,0180 | Canyon Diablo | |
| | ³³S | 0,7500 | Troilite | 0,0450045 |
| | ³⁴S | 4,2150 | (CDT) | |
| | ³⁵S | 0,0200 | | |

In der Tabelle sind die Elemente und ihre stabilen Isotope, die für die Markierung von Pigmenten oder Füllstoffen von Bedeutung sind, ihre relativen mittleren Häufigkeiten F (F = [Isotop]/[ΣIsotope]), der Name des internationalen Isotopenstandards und dessen Isotopenverhältnis R für die zwei häufigsten stabilen Isotope (R = [Isotop a]/[Isotop b]) angegeben. In der Praxis gibt es jedoch für jedes Element in den chemischen Verbindungen geringfügige örtliche und zeitliche Abweichungen von den angegebenen Häufigkeiten. Deshalb werden die genauen Isotopenkonzentrationen aller Proben als relative Differenzen zu jenen internationalen Standards in δ-Werten in Promille angegeben. Das heißt, es werden zunächst die δ-Werten für die relevanten Isotope der verschiedenen Pigmente und Füllstoffe bestimmt (beispielsweise: Isotopenverhältnis ¹⁸O/¹⁶O gegen den entsprechenden Standard V-SMOW für BaSO₄ oder Isotopenverhältnis ³⁴S³²S gegen den entsprechenden Standard CDT für ZnS), was dann als natürliches Isotopenverhältnis für diese Verbindung unter den verwendeten Bedingungen (eingesetzte Rohstoffe, Herstellbedingungen) festgesetzt wird.

Die Verwendung von stabilen Isotopen zur Authentifiezierung von Lebensmitteln wird von Ulberth Franz "Analytical Approaches for Food Authentication", Mitteilungen aus Lebensmitteluntersuchung und Hygiene, Vol.95, no.6, 2004, beschrieben.

Bei der Markierung nach dem Stand der Technik muss der Problematik Rechnung getragen werden, dass der Anteil der markierenden Isotope im zu markierenden Stoff beispielsweise durch verfahrenstechnische Schritte verringert werden kann. So können Proben, deren Herkunft identifiziert werden soll, gegebenenfalls verdünnt worden sein. Dies ist insbesondere bei Futter- und Lebensmitteln, beispielsweise Fruchtsäften, der Fall. Bei Zugabe beispielsweise von isotopenmarkiertem Wasser kann dieses durch Trocknung oder dergleichen dem zu markierenden Stoff entzogen werden. Auch nach der Verdünnung oder Trocknung muss die Konzentration von stabilem Isotop immer noch signifikant über oder unter der natürlich vorkommenden Konzentration liegen, damit ein Nachweis über die Herkunft erfolgen kann. Die Folge ist, dass entsprechend hohe Konzentrationen markierter Substanz eingesetzt werden müssen. Dies hat jedoch einen hohen Verbrauch an markierter Substanz und damit verbunden hohe Kosten zur Folge. Weiterhin können insbesondere bei Nahrungsmitteln durch unerwünscht hohe Konzentrationen an markierter Substanz toxische Nebenwirkungen auftreten, die nicht akzeptabel sind. Ist die Verdünnung beliebig hoch, so kann die Markierung gegebenenfalls nicht mehr gemessen werden.

Von messtechnischer Seite wurde dieses Problem teilweise durch die technische Lehre von DE-A-102 00 802 gelöst. Jedoch bezieht sich dieses Verfahren nur auf eine Markierung mit deuteriertem Wasser. Damit hat dieses Verfahren den Nachteil, dass es nur angewendet werden kann, wenn das eingebrachte Wasser zumindest teilweise dauerhaft im Endprodukt verbleibt. Dieses Verfahren ist also nicht auf die Markierung von anorganischen Additiven, insbesondere Pigmenten und Füllstoffen, anwendbar. Diese werden bei ihrer Herstellung und Verarbeitung mehrfach getrocknet bzw. einer thermischen Behandlung ausgesetzt, bei der eine Temperatur von 100°C oftmals überschritten wird, beispielsweise bei der Extrusion von polymeren Werkstoffen.

Aufgabe der vorliegenden Erfindung ist es, die Nachteile des Standes der Technik zu überwinden.

Insbesondere Aufgabe der vorliegenden Erfindung ist es, ein anorganisches Additiv, das dauerhaft durch ein oder mehrere schwere, stabile Isotope markiert ist, und ein Verfahren zu dessen Herstellung bereitzustellen. Eine weitere Aufgabe der Erfindung ist es, ein anorganisches Additiv bereitzustellen, dessen Gehalt des zur Markierung eingesetzten Isotops bzw. der zur Markierung eingesetzten Isotope durch thermische Behandlung nicht beeinflusst werden kann.

Erfindungsgemäß wird dieses Problem überraschenderweise durch die Merkmale des Hauptanspruchs gelöst. Vorzugsweise Ausgestaltungen finden sich in den Unteransprüchen.

Damit bei der Herstellung des Endproduktes die Markierung nicht verändert wird oder verloren geht, muss die Markierung dauerhaft bzw. fest in die chemische Verbindung des Additivs eingebaut werden. Wenn beispielsweise ein anorganisches Additiv wie TiO₂, das beispielsweise als Weißpigment eingesetzt werden kann, vor der Verwendung nur mit O¹⁸-markiertem Wasser befeuchtet wird, kann es geschehen, dass die Markierung bei der Herstellung des Endproduktes nur teilweise oder gar nicht in das Endprodukt eingebracht wird. Dieses kann bei Verwendung von markiertem Wasser immer dann geschehen, wenn ein thermischer oder Wasser entziehender Schritt im Herstellprozess des Additivs bzw. des Endproduktes unter Verwendung des Additivs enthalten ist.

Die Markierung mit schweren stabilen Isotopen muss so gewählt werden, dass der Gehalt im Endproduktes zweifelsfrei nachgewiesen werden kann (beispielsweise durch Stabil Isotop Massenspektometrie), auch wenn nur wenige Milligramm des Endproduktes zur Verfügung stehen.

Dabei wird erfindungsgemäß das Problem durch ein anorganisches Additiv gelöst, das im Vergleich zum natürlichen Vorkommen einen erhöhten (Δδ hat positve Werte) oder erniedrigten (Δδ hat negative Werte) Anteil an schweren stabilen Isotopen, beispielsweise ²H, ¹³C, ¹⁵N, ¹⁸O und/oder ³⁴S aufweist. Vorzugsweise handelt es sich bei dem anorganischen Additiv um ein Pigment oder einen Füllstoff. Beispiele für ein solches anorganisches Additiv sind TiO₂, ZnS oder BaSO₄.

Die Markierung ist fest im Additiv eingebaut. Fest eingebaut bedeutet in diesem Zusammenhang, dass für ein bestimmtes Additiv das jeweilige Δδ einen konstanten Wert hat. Darüber hinaus kann gezielt ein bestimmter Gehalt an schweren Isotopen, das heißt ein bestimmtes Δδ, eingestellt werden.

Dabei bezeichnet Δδ die Differenz der δ-Werte der mit schweren Isotopen angereicherten Verbindung und der nicht angereicherten, "natürlichen" Verbindung (beispielsweise δ³⁴S von mit ³⁴S markiertem ZnS minus Ö³⁴S von "natürlichem" ZnS).

Erfindungsgemäß liegen die Δδ-Werte zwischen 3 und 1000 ‰, bevorzugt zwischen 5 und 300 ‰ besonders bevorzugt zwischen 10 und 200 ‰, und/oder zwischen -3 und -100 ‰, besonders bevorzugt zwischen -5 und -20 ‰.

Durch den erhöhten oder erniedrigten Anteil an schweren Isotopen, beispielsweise ²H, ¹³C, ¹⁵N, ¹⁸O und/oder ³⁴S, können diese Stoffe eindeutig und dauerhaft markiert und nachgewiesen werden. Bei Stoffen, die durch zwei oder mehr Isotope markiert sind, können die zur Markierung eingesetzten Isotope alle angereichert sein oder alle abgereicht sein oder ein oder mehr Isotope können angereichert sein, während das andere Isotop bzw. die anderen Isotope abgereicht ist bzw. sind.

Die Markierung kann durch verschiedene Verfahren erfolgen, beispielsweise:
- das anorganische Additiv wird direkt bei der Herstellung durch den Einbau eines geeigneten schweren stabilen Isotops in die chemische Verbindung dauerhaft markiert.
- anorganische Additive, insbesondere Pigmente und Füllstoffe, werden oftmals mit einer anorganischen und/oder organischen Nachbehandlung versehen, insbesondere werden sie beschichtet. Durch diese Nachbehandlungen werden die Eigenschaften der Additive so verändert, dass sie für den Verwendungszweck optimal angepasst werden (Jochen Winkler, Titandioxid, Kapitel 3.4, Hannover: Vincentz, 2003; Elizabeth Reck, *Farbe&Lack,* 102. Jahrgang 3/96, S.40-48). Auch diese anorganische und/oder organische Nachbehandlung kann zur dauerhaften Markierung mit schweren stabilen Isotopen verwendet werden.
- Füllstoffe und Pigmente, wie beispielsweise TiO₂, ZnS oder BaSO₄ (anorganisch nachbehandelt oder nicht nachbehandelt), können durch gezielte Verunreinigung mit schwerlöslichen thermostabilen anorganischen Substanzen mit verändertem Isotopengehalt an ¹³C, ¹⁸O, ¹⁵N und/oder ³⁴S markiert werden. Für diese Verunreinigungen bieten sich beispielsweise schwerlösliche sauerstoffhaltige anorganische Al-, Ba-, Si-, Ti-, Zr-, Ca-, Mg-, Fe-, Zn- und/oder B-Verbindungen an. Diese anorganischen Verbindungen müssen nicht unbedingt oxidischer Natur sein, sondern können auch beispielsweise Anionen wie Hydroxide, Phosphate, Carbonate, Nitrate, Sulfide oder auch Sulfate enthalten.

Beliebige Kombinationen der genannten Verfahren sind ebenfalls möglich. Durch diese Verfahren wird erreicht, dass das anorganische Additiv dauerhaft durch schwere Isotope markiert ist.

Die durch die oben beschriebenen Verfahren markierten anorganischen Additive können auf allen Einsatzgebieten für Additive angewendet werden, beispielsweise als Pigmente und/oder Füllstoffe, wiederum beispielsweise in:
a) Kunststoffen/polymeren Werkstoffen (beispielsweise PET-Flaschen, Film/Folien, Synthese- und Chemiefasern);
b) Farben und Lacken;
c) Lebensmitteln;
d) pharmazeutische Mitteln;
e) kosmetische Mitteln;
f) Papier;
g) Gummi;
h) Glas;
i) Futtermitteln;

Gegenstand der Erfindung ist im Einzelnen:
- ein anorganisches Additiv, das durch schwere, stabile Isotope markiert ist;
- ein anorganisches Additiv, das durch schwere, stabile Isotope markiert ist, wobei die Markierung fest im Additiv eingebaut ist;
- ein anorganisches Additiv, das dauerhaft durch schwere, stabile Isotope markiert ist;
- ein anorganisches Additiv, dessen Gehalt des zur Markierung eingesetzten Isotops bzw. der zur Markierung eingesetzten Isotope durch thermische Behandlung nicht beeinflusst werden kann;
- ein anorganisches Additiv, das durch schwere, stabile Isotope markiert ist, wobei die Isotope ausgewählt sind aus ein oder mehreren von ²H, ¹³C, ¹⁵N, ¹⁸O und ³⁴S;
- ein anorganisches Additiv, das durch schwere, stabile Isotope markiert ist, wobei die Isotope angereichert sind;
- ein anorganisches Additiv, das durch schwere, stabile Isotope markiert ist, wobei die Isotope abgereichert sind.
- ein anorganisches Additiv, das durch schwere, stabile Isotope markiert ist, wobei das Additiv als solches markiert ist;
- ein anorganisches Additiv, das durch schwere, stabile Isotope markiert ist, wobei die Beschichtung des Additivs markiert ist;
- ein anorganisches Additiv, das durch schwere, stabile Isotope markiert ist, die in gezielten Verunreinigungen des Additivs enthalten sind;
- ein anorganisches Additiv, das durch schwere, stabile Isotope markiert ist, wobei die Δδ-Werte zwischen 3 und 1000 ‰, bevorzugt zwischen 5 und 300 ‰ besonders bevorzugt zwischen 10 und 200 ‰, und/oder zwischen -3 und -100 ‰, besonders bevorzugt zwischen -5 und -20 ‰ liegen;
- ein anorganisches Additiv, das durch schwere, stabile Isotope markiert ist, wobei es sich bei dem Additiv um ein Pigment und/oder einen Füllstoff handelt;
- ein anorganisches Additiv, das durch schwere, stabile Isotope markiert ist, wobei es sich beim Additiv um TiO₂, ZnS oder BaSO₄ handelt;
- ein Verfahren zur Herstellung eines markierten anorganischen Additivs;
- ein Verfahren zur Herstellung eines markierten anorganischen Additivs, wobei die Markierung fest im Additiv eingebaut wird;
- ein Verfahren zur Herstellung eines markierten anorganischen Additivs, wobei die Markierung durch ein oder mehrere schwere, stabile Isotope und/oder deren Verbindungen erfolgt;
- ein Verfahren zur Herstellung eines markierten anorganischen Additivs, wobei die Isotope ausgewählt sind aus ein oder mehreren von ²H, ¹³C, ¹⁵N, ¹⁸O und/oder ³⁴S;
- ein Verfahren zur Herstellung von markierten anorganischen Additiven, wobei die Isotope angereichert sind;
- ein Verfahren zur Herstellung eines markierten anorganischen Additivs, wobei die Isotope abgereichert sind;
- ein Verfahren zur Herstellung eines markierten anorganischen Additivs, wobei das Additiv unmittelbar bei der Herstellung durch den Einbau eines schweren stabilen Isotops in die chemische Verbindung des Additivs markiert wird;
- ein Verfahren zur Herstellung eines markierten anorganischen Additivs, wobei das Additiv bei der anorganischen und/oder organischen Nachbehandlung mit schweren stabilen Isotopen markiert wird;
- ein Verfahren zur Herstellung eines markierten anorganischen Additivs, wobei das Additiv durch gezielte Verunreinigung mit schwerlöslichen, thermostabilen anorganischen Substanzen markiert wird;
- ein Verfahren zur Herstellung eines markierten anorganischen Additivs, wobei es sich bei dem Additiv um ein Pigment und/oder einen Füllstoff handelt;
- ein Verfahren zur Herstellung eines markierten anorganischen Additivs, wobei es sich bei dem Additiv um TiO₂, ZnS oder BaSO₄ handelt;
- ein Verfahren zur Herstellung eines markierten anorganischen Additivs, wobei der ¹⁸O-Gehalt in TiO₂ oder BaSO₄ erhöht wird;
- ein Verfahren zur Herstellung eines markierten anorganischen Additivs, wobei der ³⁴S-Gehalt in ZnS oder BaSO₄ erhöht wird;
- ein Verfahren zur Herstellung eines markierten anorganischen Additivs, wobei der ¹⁸O-Gehalt in der anorganischen und/oder organischen Nachbehandlung von TiO₂, ZnS oder BaSO₄ durch ¹⁸O-markierte Oxide bzw. Hydroxide des Aluminiums, des Siliziums, des Zirkons, des Mangans und/oder des Titans erhöht wird;
- ein Verfahren zur Herstellung eines markierten anorganischen Additivs, wobei der ²H-, ¹³C-, ¹⁵N-, ¹⁸O- und/oder ³⁴S-Gehalt in der organischen Nachbehandlung durch Oberflächenbehandlung mit Polyalkoholen, Aminoverbindungen und/oder Silikonölen mit verändertem Isotopengehalt an ²H, ¹³C, ¹⁸O, ¹⁵N und/oder ³⁴S erhöht oder erniedrigt wird;
- ein Verfahren zur Herstellung eines markierten anorganischen Additivs, wobei der ²H- ¹³C- ¹⁸O-, ¹⁵N- und/oder ³⁴S-Gehalt durch gezielte Verunreinigung mit schwerlöslichen thermostabilen anorganischen Substanzen mit verändertem Isotopengehalt an ²H, ¹³C, ¹⁸O, ¹⁵N und/oder ³⁴S, insbesondere mit sauerstoffhaltigen anorganischen Al-, Si-, Ti-, Zr-, Ca-, Mg-, Fe-, Zn- und/oder B-Verbindungen, erhöht oder erniedrigt wird;
- die Verwendung des erfindungsgemäßen Additivs als Füllstoff und/oder Pigment; .
- die Verwendung des erfindungsgemäßen Additivs bei der Herstellung und Verarbeitung von Kunststoffen/polymeren Werkstoffen (PET-Flaschen, Film/Folien, Synthese- und Chemiefasern), Farben und Lacken, Lebensmitteln; pharmazeutischen Mitteln, kosmetischen Mitteln, Papier; Gummi, Glas, Futtermitteln, vorzugsweise als Pigment und/oder Füllstoff.

Die Erfindung wird durch die nachfolgenden Beispiele näher erläutert, ohne sie darauf einzuschränken:

### Beispiel 1: Herstellung von markierten anorganischen Additiven

a. Erhöhung des ¹⁸O-Gehaltes in TiO₂ oder BaS04:
   i. Herstellung von TiO₂ durch Hydrolyse von titanhaltigen Verbindungen wie Titantetrachlorid mit ¹⁸O-markiertem Wasser; die Umsetzung führt zu einem TiO₂-Produkt mit einem δ(¹⁸O)-Wert von +50 ‰; ein unmarkiertes TiO₂-Produkt weist dagegen einen δ(¹⁸O)-Wert von -2,7 ‰ auf; der Δδ-Wert beträgt also +52,7 ‰;
   ii. Herstellung von TiO₂ durch Hydrolyse von titanhaltigen Verbindungen wie Titanylsulfat-Lösung mit ¹⁸O-markiertem Wasser;
   iii. Herstellung von BaSO₄ durch Fällung aus wässriger Bariumchlorid-Lösung mit ¹⁸O-markierter Schwefelsäure; die Umsetzung führt zu einem BaSO₄-Produkt mit einem δ(¹⁸O)-Wert von +33 ‰; ein unmarkiertes BaSO₄-Produkt weist dagegen einen δ(¹⁸O)-Wert von -3 ‰ auf; der Δδ-Wert beträgt also +36 ‰.
b. Erhöhung des ³⁴S-Gehaltes in ZnS oder BaSO₄.
   i. Herstellung von ZnS durch Reaktion von ZnO mit ³⁴S-markierter Schwefelsäure zu Zinksulfat und anschließende Umsetzung mit Na₂S zu ZnS;
   ii. Herstellung von BaSO₄ durch Fällung aus wässriger Bariumchlorid-Lösung mit ³⁴S-markierter Schwefelsäure.

### Beispiel 2: Herstellung von in der Beschichtung markierten anorganischen Additiven

a. Erhöhung des ¹⁸O-Gehaltes in der anorganischen und/oder organischen Nachbehandlung von TiO₂. ZnS oder BaSO₄. Bei der anorganischen Nachbehandlung werden schwerlösliche, farblose anorganische Verbindungen auf die einzelnen Pigmentteilchen aufgefällt. Zur Oberflächenbehandlung finden beispielsweise die Oxide bzw. Hydroxide des Aluminiums, des Siliziums, des Zirkons, des Mangans und/oder des Titans Verwendung. Diese Fällungsreaktionen können unter Verwendung von ¹⁸O-markierten Rohstoffen durchgeführt werden;
b. Erhöhung des ²H, ¹³C, ¹⁵N und/oder ¹⁸O-Gehaltes in der organischen Nachbehandlung von TiO₂, ZnS oder BaSO₄. Zur Oberflächenbehandlung von Füllstoffen und Pigmenten werden beispielsweise Verbindungen wie Polyalkohole, Aminoverbindungen oder auch Silikonöle verwendet. Auch diese Verbindungen gelangen mit dem Additiv bis ins Endprodukt und können somit für die Markierung herangezogen werden.

### Beispiel 3: Herstellung von markierten Pigmenten oder Füllstoffen durch gezielte Verunreinigung:

a. Verunreinigung von TiO₂ mit ¹⁸O-markiertem ZrO₂;
b. Verunreinigung von ZnS mit ¹³C-markiertem CaCO₃;
c. Verunreinigung von BaSO₄ mit ¹⁵N und/oder ¹⁸O-markiertem MgNH₄PO₄;
d. Verunreinigung von TiO₂ mit ¹⁸O-markiertem BaSO₄; die Zugabe von mit einem Gew.-% ¹⁸O-markiertem BaSO₄ zu einem TiO₂-Pigment ergibt im verunreinigten Endprodukt einen δ(¹⁸O)-Wert von +162 ‰; die Zugabe von einem Ges.-% unmarkiertem BaSO₄ zu einem TiO₂-Pigment ergibt dagegen im verunreinigten Endprodukt einen δ(¹⁸O)-Wert von -3 ‰; der Δδ-Wert beträgt also +165 ‰;

Beliebige Kombinationen aus den Beispielen 1 bis 3 sind ebenfalls möglich.

## Patentansprüche

1. Anorganisches Additiv, das durch schwere, stabile Isotope markiert ist, **dadurch gekennzeichnet, dass** die Markierung fest im Additiv eingebaut ist.

2. Anorganisches Additiv nach Anspruch 1, **dadurch gekennzeichnet, dass** es sich um einen Füllstoff und/oder ein Pigment handelt.

3. Anorganisches Additiv nach Anspruch 1 oder 2, **dadurch gekennzeichnet, dass** es sich um TiO₂, ZnS oder BaSO₄ handelt.

4. Anorganisches Additiv nach einem der Ansprüche 1 bis 3, **dadurch gekennzeichnet, dass** die Isotope ausgewählt sind aus ein oder mehreren von ²H, ¹³C, ¹⁵N, ¹⁸O und/oder ³⁴S.

5. Anorganisches Additiv nach einem der Ansprüche 1 bis 4, **dadurch gekennzeichnet, dass** die Isotope angereichert sind.

6. Anorganisches Additiv nach einem der Ansprüche 1 bis 5, **dadurch gekennzeichnet, dass** die Isotope abgereichert sind.

7. Anorganisches Additiv nach einem der Ansprüche 1 bis 6, **dadurch gekennzeichnet, dass** das Additiv als solches markiert ist.

8. Anorganisches Additiv nach einem der Ansprüche 1 bis 7, **dadurch gekennzeichnet, dass** die Beschichtung des Additivs markiert ist.

9. Anorganisches Additiv nach einem der Ansprüche 1 bis 8, **dadurch gekennzeichnet, dass** es durch gezielte Verunreinigungen markiert ist.

10. Anorganisches Additiv nach einem der Ansprüche 1 bis 9, **dadurch gekennzeichnet, dass** die Δδ-Werte zwischen 3 und 1000 ‰, bevorzugt zwischen 5 und 300 ‰ besonders bevorzugt zwischen 10 und 200 ‰, und/oder zwischen -3 und -100 ‰, besonders bevorzugt zwischen -5 und -20 ‰ liegen

11. Verfahren zur Herstellung eines anorganischen Additivs nach einem der Ansprüche 1 bis 10, **dadurch gekennzeichnet, dass** die Markierung fest in die chemische Verbindung des Additivs eingebaut wird.

12. Verfahren nach Anspruch 11, **dadurch gekennzeichnet, dass** die anorganischen Additive direkt bei der Herstellung durch den Einbau eines geeigneten schweren stabilen Isotops in die chemische Verbindung dauerhaft markiert werden.

13. Verfahren nach Anspruch 11 oder 12 **dadurch gekennzeichnet, dass** die anorganischen Additive bei der anorganischen und/oder organischen Nachbehandlung mit schweren stabilen Isotopen dauerhaft markiert werden.

14. Verfahren nach einem der Ansprüche 11 bis 13, **dadurch gekennzeichnet, dass** die anorganischen Additive durch gezielte Verunreinigung mit schwerlöslichen thermostabilen anorganischen Substanzen dauerhaft markiert werden.

15. Verfahren nach einem der Ansprüche 11 bis 14, **dadurch gekennzeichnet, dass** der ¹⁸O-Gehalt in TiO₂ oder BaSO₄ erhöht wird.

16. Verfahren nach einem der Ansprüche 11 bis 15, **dadurch gekennzeichnet, dass** der ³⁴S-Gehalt in ZnS oder BaSO₄ erhöht wird.

17. Verfahren nach einem der Ansprüche 11 bis 16, **dadurch gekennzeichnet, dass** der ¹⁸O-Gehalt in der anorganischen und/oder organischen Nachbehandlung von TiO₂, ZnS oder BaSO₄ durch ¹⁸O-markierte Oxide bzw. Hydroxide des Aluminiums, des Siliziums, des Zirkons, des Mangans und/oder des Titans erhöht wird.

18. Verfahren nach einem der Ansprüche 11 bis 17, **dadurch gekennzeichnet, dass** der ²H-, ¹³C, ¹⁵N-, ¹⁸O- und/oder ³⁴S-Gehalt in der organischen Nachbehandlung durch Oberflächenbehandlung mit Polyalkoholen, Aminoverbindungen und/oder Silikonölen mit verändertem Isotopen gehalt an ¹³C, ¹⁸O, ¹⁵N und/oder ³⁴S erhöht wird.

19. Verfahren nach einem der Ansprüche 11 bis 18, **dadurch gekennzeichnet, dass** der ²H-, ¹³C-, ¹⁸O-, ¹⁵N- und/oder ³⁴S-Gehalt durch gezielte Verunreinigung mit schwerlöslichen thermostabilen anorganischen Substanzen mit verändertem Isotopengehalt an ²H, ¹³C, ¹⁸O, ¹⁵N und/oder ³⁴S, insbesondere mit sauerstoffhaltigen anorganischen Al-, Si-, Ti-, Zr-, Ca-, Mg-, Fe-, Zn- und/oder B-Verbindungen, erhöht wird.

20. Verwendung des anorganischen Additivs nach einem der Ansprüche 1 bis 10 als Pigment und/oder Füllstoff.

21. Verwendung des anorganischen Additivs nach einem der Ansprüche 1 bis 10 bei der Herstellung und Verarbeitung von Kunststoffen/polymeren Werkstoffen, Farben und Lacken, Lebensmitteln; pharmazeutischen Mitteln, kosmetischen Mitteln, Papier; Gummi, Glas, Futtermitteln, vorzugsweise als Pigment und/oder Füllstoff.

## Claims

1. Inorganic additive that is marked by heavy stable isotopes, **characterised in that** the marking is fixedly incorporated in the additive.

2. Inorganic additive according to claim 1, **characterised in that** it is a filler and/or a pigment.

3. Inorganic additive according to claim 1 or 2, **characterised in that** it is TiO₂, ZnS or BaSO₄.

4. Inorganic additive according to one of claims 1 to 3, **characterised in that** the isotopes are selected from one or a plurality of ²H, ¹³C, ¹⁵N, ¹⁸O and/or ³⁴S .

5. Inorganic additive according to one of claims 1 to 4, **characterised in that** the isotopes are enriched.

6. Inorganic additive according to one of claims 1 to 5, **characterised in that** the isotopes are depleted.

7. Inorganic additive according to one of claims 1 to 6, **characterised in that** the additive is marked as such.

8. Inorganic additive according to one of claims 1 to 7, **characterised in that** the coating of the additive is marked.

9. Inorganic additive according to one of claims 1 to 8, **characterised in that** it is marked by specific contaminations.

10. Inorganic additive according to one of claims 1 to 9, **characterised in that** the Δδ-values lie between 3 and 1000 ‰, preferably between 5 and 300 ‰, particularly preferably between 10 and 200 ‰, and/or between -3 and -100 ‰_{,} particularly preferably between -5 and -20 ‰.

11. A method for the production of an inorganic additive according to one of claims 1 to 10, **characterised in that** the marking is fixedly incorporated in the chemical compound of the additive.

12. A method according to claim 11, **characterised in that** the inorganic additives are permanently marked directly during production by the incorporation of a suitable heavy stable isotope in the chemical compound.

13. A method according to claim 11 or 12, **characterised in that** the inorganic additives are permanently marked during inorganic and/or organic aftertreatment with heavy stable isotopes.

14. A method according to one of claims 11 to 13, **characterised in that** the inorganic additives are permanently marked by specific contamination with thermostable inorganic substances that are difficult to dissolve.

15. A method according to one of claims 11 to 14, **characterised in that** the ¹⁸O-content in TiO₂ or BaSO₄ is increased.

16. A method according to one of claims 11 to 15, **characterised in that** the ³⁴S-content in ZnS or BaSO₄ is increased.

17. A method according to one of claims 11 to 16, **characterised in that** the ¹⁸O-content is increased in the inorganic and/or organic aftertreatment of TiO₂, ZnS or BaSO₄ by ¹⁸O-marked oxides or hydroxides of aluminium, silicon, zircon, manganese and/or titanium.

18. A method according to one of claims 11 to 17, **characterised in that** the ²H-, ¹³C-, ¹⁵N-, ^{1B}O- and/or ³⁴S-content is increased in organic aftertreatment by means of surface treatment with polyalcohols, amino compounds and/or silicone oils with a changed isotope content of ¹³C, ¹⁸O, ¹⁵N and/or ³⁴S.

19. A method according to one of claims 11 to 18, **characterised in that** the ²H-, ¹³C-, ¹⁸O-, ¹⁵N- and/or ³⁴S-content is increased by specific contamination with thermostable inorganic substances that are difficult to dissolve with a changed isotope content of ²H, ¹³C, ¹⁸O, ¹⁵N and/or ³⁴S, in particular with oxygen-containing inorganic Al-, Si-, Ti-, Zr-, Ca-, Mg-, Fe-, Zn- and/or B-compounds.

20. Use of the inorganic additive according to one of claims 1 to 10 as a pigment and/or filler.

21. Use of the inorganic additive according to one of claims 1 to 10 in the production and processing of plastics/polymer materials, paints and lacquers, foodstuffs; pharmaceutical agents, cosmetic agents, paper; rubber, glass, feedstuffs, preferably as a pigment and/or filler.

## Revendications

1. Adjuvant inorganique marqué à l'aide d'isotopes lourds stables, **caractérisé en ce que** le marquage est solidement incorporé dans l'adjuvant.

2. Adjuvant inorganique conforme à la revendication 1, **caractérisé en ce qu'**il s'agit d'une charge et/ou d'un pigment.

3. Adjuvant inorganique conforme à la revendication 1 ou 2, **caractérisé en ce qu'**il s'agit de dioxyde de titane TiO₂, de sulfure de zinc ZnS ou de sulfate de baryum BaSO₄.

4. Adjuvant inorganique conforme à l'une des revendications 1 à 3, **caractérisé en ce que** les isotopes sont choisis, un ou plusieurs, parmi les suivants : ²H, ¹³C, ¹⁵N, ¹⁸O et/ou ³⁴S.

5. Adjuvant inorganique conforme à l'une des revendications 1 à 4, **caractérisé en ce qu'**il est enrichi en isotope(s).

6. Adjuvant inorganique conforme à l'une des revendications 1 à 5, **caractérisé en ce qu'**il est appauvri en isotope(s).

7. Adjuvant inorganique conforme à l'une des revendications 1 à 6, **caractérisé en ce que** c'est l'adjuvant lui-même qui est marqué.

8. Adjuvant inorganique conforme à l'une des revendications 1 à 7, **caractérisé en ce que** c'est l'enrobage de l'adjuvant qui est marqué.

9. Adjuvant inorganique conforme à l'une des revendications 1 à 8, **caractérisé en ce qu'**il est marqué par contaminations orientées.

10. Adjuvant inorganique conforme à l'une des revendications 1 à 9, **caractérisé en ce que** les valeurs de Δδ se situent entre 3 et 1000 ‰, de préférence entre 5 et 300 ‰ et surtout entre 10 et 200 ‰, et/ou entre -3 et -100 ‰ et surtout entre -5 et -20 ‰.

11. Procédé de préparation d'un adjuvant inorganique conforme à l'une des revendications 1 à 10, **caractérisé en ce que** le marquage est solidement incorporé dans le composé chimique de l'adjuvant.

12. Procédé conforme à la revendication 11, **caractérisé en ce que** l'on marque durablement l'adjuvant inorganique, lors même de sa préparation, en incorporant un isotope lourd stable approprié dans le composé chimique.

13. Procédé conforme à la revendication 11 ou 12, **caractérisé en ce que** l'on marque durablement l'adjuvant inorganique lors d'un post-traitement inorganique et/ou organique avec un isotope lourd stable approprié.

14. Procédé conforme à l'une des revendications 11 à 13, **caractérisé en ce que** l'on marque durablement l'adjuvant inorganique par contamination orientée avec des substances inorganiques thermostables et peu solubles.

15. Procédé conforme à l'une des revendications 11 à 14, **caractérisé en ce que** l'on élève la teneur en isotope ¹⁸O de TiO₂ ou BaSO₄.

16. Procédé conforme à l'une des revendications 11 à 15, **caractérisé en ce que** l'on élève la teneur en isotope ³⁴S de ZnS ou BaSO₄.

17. Procédé conforme à l'une des revendications 11 à 16, **caractérisé en ce que** l'on élève la teneur en isotope ¹⁸O, lors d'un post-traitement inorganique et/ou organique de TiO₂, ZnS ou BaSO₄, au moyen d'oxydes ou hydroxydes, marqués à l'oxygène-18, d'aluminium, de silicium, de zirconium, de manganèse et/ou de titane.

18. Procédé conforme à l'une des revendications 11 à 17, **caractérisé en ce que** l'on élève la teneur en isotope(s) ²H, ¹³C, ¹⁵N, ¹⁸O et/ou ³⁴S, lors d'un post-traitement organique, en effectuant un traitement de surface avec des polyalcools, des composés aminés et/ou des huiles de silicone à teneurs modifiées en isotope(s) ²H, ¹³C, ¹⁵N, ¹⁸O et/ou ³⁴S.

19. Procédé conforme à l'une des revendications 11 à 18, **caractérisé en ce que** l'on élève la teneur en isotope(s) ²H, ¹³C, ¹⁵N, ¹⁸O et/ou ³⁴S par contamination orientée avec des substances inorganiques thermostables et peu solubles à teneurs modifiées en isotope(s) ²H, ¹³C, ¹⁵N, ¹⁸O et/ou ³⁴S, en particulier avec des composés inorganiques oxygénés d'aluminium, de silicium, de titane, de zirconium, de calcium, de magnésium, de fer, de zinc et/ou de bore.

20. Utilisation d'un adjuvant inorganique conforme à l'une des revendications 1 à 10 en qualité de pigment et/ou de charge.

21. Utilisation d'un adjuvant inorganique conforme à l'une des revendications 1 à 10 dans la fabrication et la mise en oeuvre de matières plastiques ou matériaux polymères, de colorants et vernis, de produits alimentaires, de produits pharmaceutiques, de produits cosmétiques, de papier, de caoutchouc, de verre ou d'aliments pour animaux, de préférence en qualité de pigment et/ou de charge.
